# EUROPEAN PATENT APPLICATION

(11) **EP 2 082 775 A1**
(43) Date of publication of application: **29.07.2009**
(21) Application number: 08150680.0
(22) Date of filing: 25.01.2008
(51) Int. Cl.: A61M 16/08

(54) **Filter**

(71) Applicant: Flodins Filter Aktiebolag, 453 29 Lysekil (SE)
(72) Inventor: Stjerfelt, Claes, 450 33, Grundsund (SE); Grundén, Inger, 453 37, Lysekil (SE); Weiselblad, Anders, 223 64, Stockholm (SE)
(74) Representative: Bergström, Johan Erik

(57) **Abstract**

Filter to be used in the expiratory branch of a ventilator, comprising a housing (10) arranged with an inlet passage (12) and an outlet passage (14), a main filter element (28), a dividing element (22) arranged inside said housing, forming two compartments, one in communication with the inlet passage and one in communication with the outlet passage, and wherein said dividing element is arranged with an opening (24), in which the main filter element is placed, which compartments are capable of collecting condensate on either side of said main filter element without contact with said main filter.

## Description

### TECHNICAL AREA

The present invention relates to a filter and in particular a filter arranged between a patient and a ventilator, capable of cleaning respiratory air.

### TECHNICAL BACKGROUND

The use of ventilators in hospitals and the like treatment facilities is very common in order to aid the patient's respiration or even replace the ordinary respiration of the patient. The patient's lungs are thus connected to a ventilator via hoses forcing air into and out of the lungs. There are commonly two hoses from the ventilator, one for expiratory air and one for inspiratory air. These are usually joined in a Y-connection near the patient.

In order to provide comfort to the patient, the inspiratory air has to be pre- heated by the ventilator, a so called single heat system. Some ventilator systems also have heating on the expiratory limb, so-called dual heat systems.

Further, it has become more and more common to use nebulizers in connection with ventilators for administering medicament to the patient. Nebulizers are capable of aerosolizing medicament, i.e. creating very small droplets that are inspired by the patient, thereby reaching the lungs, where the medicament is further distributed into the body of the patient.

In order to prevent that the ventilator becomes contaminated by viruses and/or bacteria exhaled by the patient, a filter is arranged in the expiratory branch. Often the filter is arranged close to the ventilator, a so-called machine side filter.

The exhaled air contains quite a lot of moist, to a large extent due to that moist is added in the ventilator system in order to aid the patient. In a single heat system this moist will condensate in the expiratory branch, whereby water traps often are arranged in the branch. However, some moist reaches the filter and will condense on the patient side of the filter. The condensate will wet the filter material causing the filter to function less adequately. This will cause an increase in resistance in the filter and thus a pressure drop that is negative for the patient.

In order to handle this situation, the applicant of the present patent application has developed a filter having a compartment under the filter element, in which the condensate is collected, whereby it is kept out of contact with the filter element, thereby prolonging the life of the filter.

The dual heat systems have been developed in order to reduce the amount of condensate in the expiratory branch, which condensate could be contaminated and could lead to an increased flow resistance. However, this just moves the problem with condensation. The filter element is capable of letting through the moist, but when the moist has passed the filter element, it will condense because this side of the filter is not heated. Thus, the condensate will enter the filter from this side and wet the filter element, with a reduced function as a consequence.

A further problem is the frequent use of nebulizers for administering medicament to the patient. The very small droplets should be inhaled into the lungs of the patient but a certain amount of the droplets will not be inhaled properly but will instead follow the exhaled air in the expiratory branch. A large amount of these droplets will then eventually be stuck in the filter element, severely affecting the function of the filter by clogging.

Recently a specially designed filter has been released on the market, having a wall or fixed surface facing the inlet of the filter, which surface is covered with an absorbing material. The incoming air is thus forced to deflect around the surface while the heavier medicament droplets get caught on the absorbing material. The design of this filter is however not optimal regarding keeping condensate out of contact with the filter element.

As described a few solutions have been presented to handle certain specific problems regarding filters for ventilators, but there is room for improvements regarding handling all types of problem with one filter concept.

### BRIEF DESCRIPTION OF THE INVENTION

The aim of the present invention is to remedy the above mentioned drawbacks of the state of the art technology. This aim is obtained by a filter with the features of the independent patent claim 1.

Preferable embodiments of the present invention are the subject of the dependent claims.

According to a main aspect of the present invention, it is characterised by a filter to be used in the expiratory branch of a ventilator, comprising a housing arranged with an inlet passage and an outlet passage, a main filter element, a dividing element arranged inside said housing, forming two compartments, one in communication with the inlet passage and one in communication with the outlet passage, and wherein said dividing element is arranged with a opening, in which the main filter element is placed, which compartments are capable of collecting condensate on either side of said main filter element without contact with said main filter.

According to a further aspect of the invention, said dividing element is in the form of a wall and wherein said main filter element is attached around the edges of said opening.

According to another aspect of the invention, it further comprises a filter member arranged adjacent, generally transversal, to the inlet passage comprising an air permeable, but liquid absorbing, material for collecting medicament droplets in the air stream through said filter.

According to a further aspect of the invention, it further comprises a holder means for said filter member creating an air passage around said filter member.

According to another aspect of the invention, said dividing element further comprises fixation means capable of fixating said filter element in said holder means.

There are several advantages with the filter according to the present invention. Due to the design with two compartments, one on each side of the main bacteria/virus filter element condensate on both sides of the filter can be managed without the risk of the filter element becoming wet by the condensate. Thus, the filter according to the present invention is capable of being used for both single heat systems as well as dual heat systems.

The dual compartment design is preferably obtained by a divider arranged inside the filter housing and having an opening where the main filter element is attached. Thus, condensation of moist on both sides of the filter element is handled by the divider that together with the housing forms the compartments.

A further advantage with the filter according to the present invention is that it also is capable of handling medicament droplets that otherwise tend to clog the filter element. Accordingly a filter member is arranged at the inlet in the airflow. The filter member admits air through but catches medicament droplets and preferably absorbs them. The filter member is further placed such that there is an air passage around it so that the air can still flow through the filter even if the filter member is becoming saturated with medicament. The filter member will further improve the spreading of the air inside the filter housing, whereby the surface of the filter element is used more optimal.

In all, the filter according to the present invention handles all types of cases regarding filtering in connection with ventilators. There is thus no need for specially designed filters for a specific type of ventilator equipment and/or if nebulized medicament is administered via the respiratory branches of the ventilator.

These and other aspects of and advantages with the present invention will become apparent from the following detailed description and from the accompanying drawings.

### BRIEF DESCRIPTION OF THE DRAWINGS

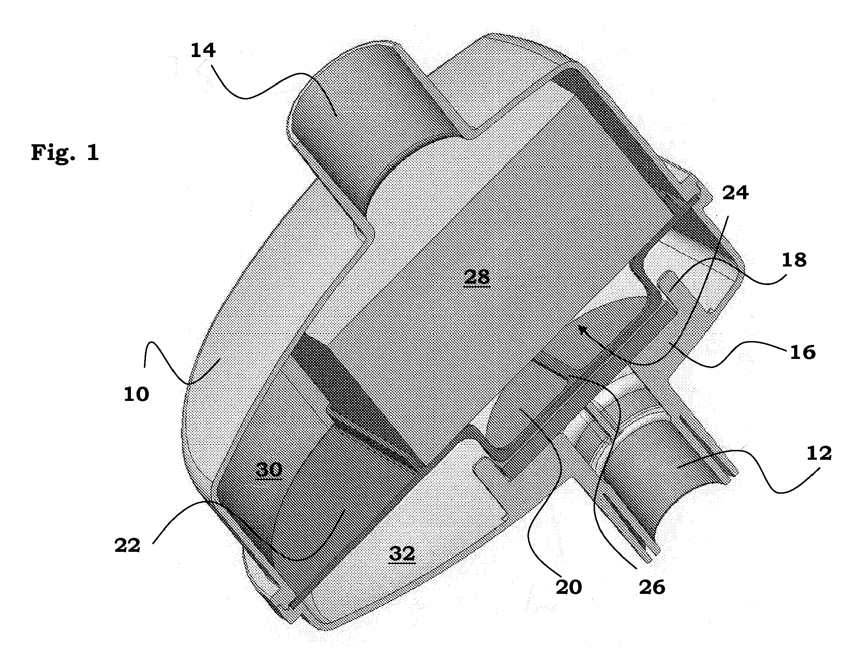

In the following detailed description of the invention, reference will be made to the accompanying drawings, of which
- Fig. 1: is a cross-sectional view in perspective of a filter according to the present invention and

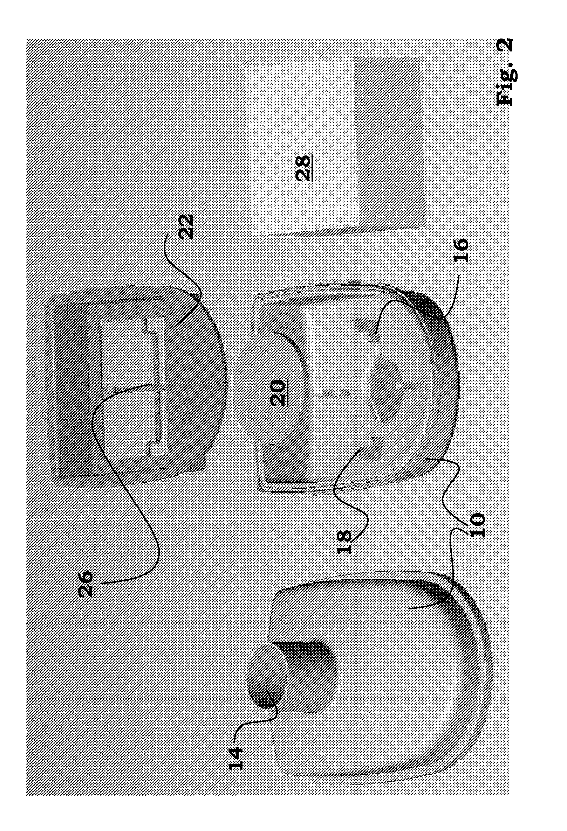

- Fig. 2: is an exploded view of the filter according to Fig. 1.

### DETAILED DESCRIPTION OF THE INVENTION

The filter shown in the figures comprises a housing 10, preferably made of plastic in two halves. The housing is arranged with an air inlet pipe connection 12 and an air outlet pipe connection 14, positioned generally opposite each other in the shown embodiment.

Around the inlet passage a number of spacer pieces 16 are attached, in the embodiment shown as four plate-like members, each arranged with a fixation part 18. On these spacer pieces a filter member 20 is placed, with a circular shape. The fixation part 18 of the spacer pieces 16 ensure that the filter member 20 is held in the proper position. The filter member is made of a suitable absorbing, yet air permeable, material. This filter member has a structure that will catch any larger droplets, like medicament droplets, while letting air through. Of course, some air will deflect around the filter member, in particular when the filter member becomes saturated with medicament because of the passage created around the filter member by the spacer pieces.

The filter further comprises a divider/holder 22. The divider/holder has a wall-like shape that preferably is attached between the two halves of the housing, thereby dividing the interior of the hosing in two parts. The divider/holder is further arranged with a central opening 24 positioned behind the filter member as seen in the direction of the air flow. In the opening, a holding structure 26 is arranged, consisting of a cross protruding towards, and in contact with, the back side of the filter member, thereby holding the filter member in the proper position.

A main viral/ bacteria filter element 28 is attached to the divider around the opening on the upstream side, completely covering the passage. The housing is designed such in relation to the main filter element that there is a space between the wall of the housing and the main filter element in the area of the outlet pipe connection. The housing is further designed such that two compartments 30, 32 are formed together with the wall of the divider/holder.

The filter according to the invention is connected to a ventilator in the expiratory branch. As described above, the filter member 20 will take care of any medicament droplets that are transported by the air stream, which air stream can pass through and/ or deflect around the filter member. However, the larger, heavier medicament drops will not deflect around the filter member, but will be caught by it. Even though the filter member over time will contain quite a lot of medicament, the air is still capable of passing through the filter because of the passage created by the spacer pieces.

The air then passes through the main filter element 28, which is capable of caching bacteria and viruses, such as HEPA or ULPA filters. The main filter element could be made of any material that is capable of collecting these, such as fibre glass or PTFE, just to mention a few.

When the filter is used in a single heat system the condensate is caught in the compartment 32 on the patient side of the filter and can thus not affect the main filter element in any negative ways.

When the filter is used in a dual heat system the moist will condense on the machine side of the filter, and in this situation the condensate is caught in the compartment 30 on the machine side of the filter.

Thus, as can be appreciated, the filter according to the present invention is capable of handling different types of situations in connection with use of ventilators.

It is to be understood that the embodiment described above and shown in the drawing is to be regarded as a non-limiting example of the invention and that it can be modified in many ways within the scope of the patent claims.

## Claims

1. Filter to be used in the expiratory branch of a ventilator, comprising
- a housing arranged with an inlet passage and an outlet passage,
- a main filter element,
- a dividing element arranged inside said housing, forming two compartments, one in communication with the inlet passage and one in communication with the outlet passage, and wherein said dividing element is arranged with a opening, in which the main filter element is placed,
- which compartments are capable of collecting condensate on either side of said main filter element without contact with said main filter.

2. Filter according to claim 1, wherein said dividing element is in the form of a wall and wherein said main filter element is attached to the edges of said opening.

3. Filter according to claim 1, wherein it further comprises a filter member arranged adjacent, generally transversal, to the inlet passage comprising an air permeable, but liquid absorbing material for collecting medicament droplets in the air stream through said filter.

4. Filter according to claim 3, wherein it further comprises a holder means for said filter member creating an air passage around said filter member.

5. Filter according to claim 4, wherein said dividing element further comprises fixation means capable of fixating said filter element in said holder means.
